# EUROPEAN PATENT APPLICATION

(11) **EP 3 488 884 A1**
(43) Date of publication of application: **29.05.2019**
(21) Application number: 18207659.6
(22) Date of filing: 21.11.2018
(51) Int. Cl.: A61M 1/16

(54) **DIALYSIS SYSTEM OF INTEGRATED MANIFOLDS**

(30) Priority: 22.11.2017 US 201762589959 P; 02.10.2018 US 201816149176
(71) Applicant: Medtronic, Inc., Minneapolis, Minnesota 55432 (US)
(72) Inventor: CHAPMAN, Paul R., Lutz, FL 33548 (US); GOMES, Carl Wilbert, Parrish, FL 34219 (US)
(74) Representative: Maschio, Antonio

(57) **Abstract**

The invention relates to a system of integrated manifolds for use in dialysis. The system of integrated manifolds can include two or more manifolds connected by fluid lines, the manifolds forming at least a portion of a dialysate flow path. In certain embodiments, valves, sensors, and/or pumps can be included. A controller can control the valves and pumps to pump fluid through the manifolds in a specified flow path.

## Description

### CROSS-REFERENCE TO RELATED APPLICATION

This application claims the benefit of and priority to U.S. Provisional Patent Application No. 62/589,959 filed November 22, 2017, the entire disclosure of which is incorporated by reference herein.

### FIELD OF THE INVENTION

The invention relates to a system of integrated manifolds for use in dialysis. The system of integrated manifolds can include two or more manifolds connected by fluid lines, the manifolds forming at least a portion of a dialysate flow path. In certain embodiments, valves, sensors, and/or pumps can be included. A controller can control the valves and pumps to pump fluid through the manifolds in a specified flow path.

### BACKGROUND

Sorbent dialysis involves regeneration of a dialysate such that spent dialysate can be reused upon proper regeneration. A dialysate flow path is needed to direct the dialysate from an outlet of a dialyzer to an inlet of the dialyzer, as well as to remove toxins and solutes from the dialysate and add additional solutes necessary for reuse. Known dialysate flow paths use a system of fluid lines to direct dialysate in the flow path. The fluid lines are susceptible to twisting, blockage, or wearing out, interrupting dialysis treatment. Hence, there is a need for systems and methods that can use multiple integrated fluid manifolds to form portions of the dialysate flow path without excessive use of flexible fluid lines. The integrated manifolds reduce the chances of blockages in the dialysate flow path, while at the same time decrease the total space requirements for the system by replacing multiple fluid lines with a single manifold. There is a further need for systems and methods that can selectively direct fluid in a specified pathway throughout the dialysate flow path based on fluid parameters of the dialysate or mode of usage.

### SUMMARY OF THE INVENTION

The first aspect of the invention relates to a system of integrated manifolds. In any embodiment, the system can comprise a dialysate flow path; the dialysate flow path fluidly connectable to a dialyzer inlet and a dialyzer outlet; the dialysate flow path comprising at least two manifolds; the at least two manifolds each comprising at least one internal conduit fluidly connecting a manifold inlet and a manifold outlet; a first fluid line fluidly connecting the dialyzer inlet to a first manifold; a second fluid line fluidly connecting the dialyzer outlet to a second manifold; and at least one fluid line fluidly connecting a manifold outlet of a least one manifold to a manifold inlet of at least one other manifold.

In any embodiment, the dialysate flow path can comprise at least three manifolds.

In any embodiment, at least one manifold can comprise at least one valve fluidly connecting the at least one internal conduit to either a first and second manifold outlet or a first and second manifold inlet.

In any embodiment, at least one valve can be in communication with a controller; the controller controlling the at least one valve to selectively direct fluid through the manifold from a specified inlet to a specified outlet.

In any embodiment, at least one manifold can comprise at least a first manifold outlet and a second manifold outlet fluidly connected to the at least one internal conduit; and at least one valve fluidly connecting the at least one internal conduit to the first manifold outlet and the second manifold outlet.

In any embodiment, at least one manifold can comprise at least a first manifold inlet and a second manifold inlet fluidly connected to the at least one internal conduit.

In any embodiment, at least one manifold can comprise at least a first internal conduit and a second internal conduit; wherein either both the first internal conduit and the second internal conduit are fluidly connected to a manifold inlet, or wherein both the first internal conduit and the second internal conduit are fluidly connected to a manifold outlet.

In any embodiment, the first internal conduit can be fluidly connected to the dialyzer inlet; and the second internal conduit can be fluidly connected to the dialyzer outlet.

In any embodiment, at least one manifold can comprise at least one sensor.

In any embodiment, the at least one sensor can be selected from the group consisting of: a pressure sensor, a conductivity sensor, a temperature sensor, a flow sensor, and combinations thereof.

In any embodiment, the dialysate flow path can comprise at least one dialysate pump external to a manifold.

In any embodiment, the dialysate flow path can comprise a sorbent cartridge external to a manifold.

In any embodiment, all valves within the dialysate flow path can be positioned within one or more manifolds.

In any embodiment, the system can comprise an ultrafiltrate reservoir fluidly connected to the dialysate flow path; the ultrafiltrate reservoir external to the at least two manifolds.

In any embodiment, the dialysate flow path can comprise a water source fluidly connected to the dialysate flow path; the water source external to the at least two manifolds.

The features disclosed as being part of the first aspect of the invention can be in the first aspect of the invention, either alone or in combination.

The second aspect of the invention relates to a method of using the system of integrated manifolds of the first aspect of the invention. In any embodiment, the method can comprise pumping a dialysate from the dialyzer outlet through a first manifold; pumping the dialysate from the first manifold through a second manifold; and pumping the dialysate from the second manifold to the dialyzer inlet.

In any embodiment, the method can comprise the step of controlling one or more valves within at least one manifold to selectively direct fluid from a specified manifold inlet to a specified manifold outlet.

In any embodiment, the step of controlling one or more valves can be performed by a controller in electronic communication with the one or more valves.

In any embodiment, the method can comprise the step of determining at least one fluid parameter of the dialysate with at least one sensor located within at least one manifold.

In any embodiment, the method can comprise the step of controlling one or more valves within at least one manifold to selectively direct fluid from a specified manifold inlet to a specified manifold outlet based on the at least one fluid parameter.

In any embodiment, the at least one fluid parameter can comprise a conductivity.

In any embodiment, the method can comprise the step of controlling at least one pump to direct a fluid from a concentrate source to the at least one internal conduit based on the at least one fluid parameter.

In any embodiment, the method can comprise the step of pumping a portion of the dialysate to an ultrafiltrate reservoir; wherein the ultrafiltrate reservoir is external to the manifolds.

In any embodiment, the method can comprise the step of pumping water from a water source to the dialysate flow path; wherein the water source is external to the manifolds.

The features disclosed as being part of the second aspect of the invention can be in the second aspect of the invention, either alone or in combination.

### BRIEF DESCRIPTION OF THE DRAWINGS

FIG. 1 is a non-limiting flow path having two integrated manifolds.
FIG. 2 is a non-limiting flow path having three integrated manifolds.

### DETAILED DESCRIPTION OF THE INVENTION

Unless defined otherwise, all technical and scientific terms used generally have the same meaning as commonly understood by one of ordinary skill in the art.

The articles "a" and "an" are used to refer to one or to over one (*i.e*., to at least one) of the grammatical object of the article. For example, "an element" means one element or over one element.

The terms "communicate" and "communication" include, but are not limited to, the connection of system electrical elements, either directly or remotely, for data transmission among and between said elements. The terms also include, but are not limited to, the connection of system fluid elements enabling fluid interface among and between said elements.

The term "comprising" includes, but is not limited to, whatever follows the word "comprising." Use of the term indicates the listed elements are required or mandatory but that other elements are optional and may be present.

A "concentrate source" is a source of one or more solutes. The concentrate source can have one or more solutes with a solute concentration greater than the solute concentration to be used for dialysis. The concentrate in the concentrate source can also be lower than the solute concentration generally used in dialysis for generation of low concentration dialysate.

The term "conductivity" refers to the inverse of the electrical resistance of a fluid.

The term "conductivity sensor" refers to any component capable of measuring the electrical conductance or the electrical resistance of a fluid.

The term "consisting of' includes and is limited to whatever follows the phrase "consisting of." The phrase indicates the limited elements are required or mandatory and that no other elements may be present.

The term "consisting essentially of' includes whatever follows the term "consisting essentially of' and additional elements, structures, acts or features that do not affect the basic operation of the apparatus, structure or method described.

A "controller" can refer to a device which monitors and affects the operational conditions of a given system. The operational conditions are typically referred to as output variables of the system wherein the output variables can be affected by adjusting certain input variables.

The terms "control," "controlling," or "controls" can refer to the ability of one component to direct the actions of a second component.

The term "determining" or "to determine" can refer to measuring any parameter or variable. The parameter or variable can relate to any state or value of a system, component, fluid, gas, or mixtures of one or more gases or fluids.

The term "dialysate flow path" refers to any portion of a fluid pathway that conveys a dialysate and is configured to form at least part of a fluid circuit for hemodialysis, hemofiltration, ultrafiltration, hemodiafiltration or ultrafiltration. Optionally, the dialysate flow path can contain priming fluid during a priming step or cleaning fluid during a cleaning step.

A "dialysate pump" is a component used to move fluid or gas throughout a dialysate flow path.

A "dialyzer inlet" is a portion of a dialyzer through which dialysate enters the dialyzer.

A "dialyzer outlet" is a portion of a dialyzer through which dialysate exits the dialyzer.

The term "external" refers to any area outside of a specified component.

The term "flow sensor" refers to a device for measuring a volume of a fluid or gas moved through a flow path per unit of time.

A "fluid line" can refer to a tubing or conduit through which a fluid or fluid containing gas can pass. The fluid line can also contain air during different modes of operation such as cleaning or purging of a line.

A "fluid parameter" is any sensed characteristic of a fluid, including temperature, pressure, concentration, color, conductivity, or any other characteristic.

The term "fluidly connectable," "fluidly connect," "for fluid connection," and the like, can refer to the ability of providing for the passage of fluid, gas, or a combination thereof, from one point to another point. The two points can be within or between any one or more of compartments, modules, systems, components, and rechargers, all of any type. The connection can optionally be disconnected and then reconnected.

The term "integrated manifolds" refers to two or more manifolds, each containing at least one internal conduit that can be fluidly connected to form part of a fluid flow path.

An "internal conduit" can refer to a fluid pathway partially or entirely inside a manifold.

A "manifold" is a component having at least one inlet and one outlet fluidly connected to an internal conduit. Fluid can enter the manifold through the inlet, travel through internal conduit, and exit the manifold through the outlet. Optionally, the manifold can have multiple inlets and/or multiple outlets fluidly connected to one or more internal conduits.

A "manifold inlet" is a portion of a manifold through which fluid or gas can enter the manifold.

A "manifold outlet" is a portion of a manifold through which fluid or gas can exit the manifold.

The term "portion" refers to all or part of any type of object. In certain embodiments, the term can be used to describe part of a dialysate in a dialysate flow path.

The term "pressure sensor" can refer to a device or any suitable component for measuring the pressure of a gas or fluid in a vessel, container, or fluid line.

The term "pump" can refers to any device that causes the movement of fluids, gases, or combinations thereof, by applying force of any type including suction or pressure.

The terms "pumping" or to "pump" refer to moving a fluid or gas through a flow path with a pump.

"Selectively directing fluid" or to "selectively direct fluid" means to cause a fluid, gas, or combinations thereof to move in a specified flow path.

A "sensor" is a component capable of determining one or more states of one or more variables in a system.

The terms "sorbent cartridge" and "sorbent container" can refer to a cartridge containing one or more sorbent materials for removing specific solutes from solution, such as urea. The term "sorbent cartridge" does not require the contents in the cartridge be sorbent based, and the contents of the sorbent cartridge can be any contents that can remove waste products from a dialysate. The sorbent cartridge may include any suitable amount of one or more sorbent materials. In certain instances, the term "sorbent cartridge" can refer to a cartridge which includes one or more sorbent materials in addition to one or more other materials capable of removing waste products from dialysate. "Sorbent cartridge" can include configurations where at least some materials in the cartridge do not act by mechanisms of adsorption or absorption. In any embodiment, a system may include a number of separate cartridges which can be physically separated or interconnected wherein such cartridges can be optionally detached and reattached as desired.

The term "specified" as used in relation to a component is meant to distinguish two similar components and is not intended to describe the structure or function of the component being described as "specified."

The term "temperature sensor" refers to a device for measuring the temperature of a gas or liquid in a vessel, container, or fluid line.

An "ultrafiltrate reservoir" is a container into which fluids, gases, or combinations thereof, can be pumped from a dialysate flow path.

A "valve" refers to a device capable of directing the flow of fluid or gas by opening, closing or obstructing one or more pathways to allow the fluid or gas to travel in a path. One or more valves configured to accomplish a desired flow can be configured into a "valve assembly."

The term "water source" refers to any source from which potable or non-potable water can be obtained.

### System of Integrated Manifolds

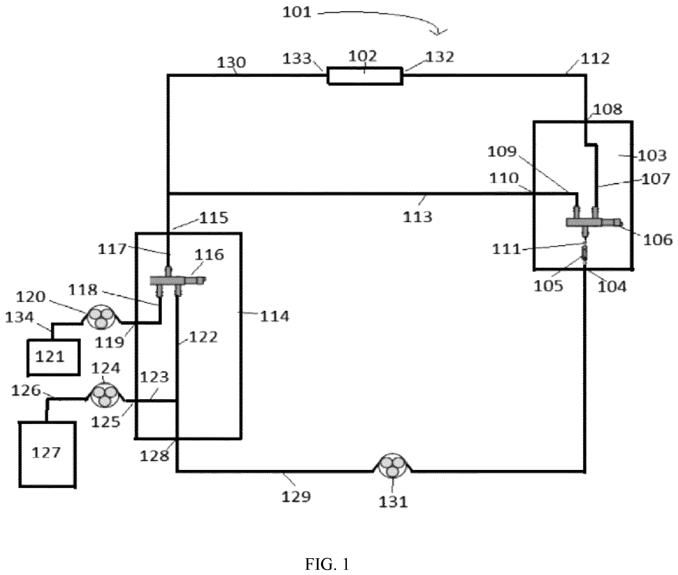

FIG. 1 illustrates a non-limiting embodiment of a dialysate flow path **101** with two integrated manifolds. The dialysate flow path **101** includes a fluid line **112** fluidly connected to a dialyzer inlet **132** of dialyzer **102**. Dialysate is pumped through the dialyzer **102** on a first side of a dialyzer membrane, while blood is pumped through an extracorporeal flow path (not shown) and through the dialyzer **102** on a second side of the dialyzer membrane. Solutes in the blood pass through the dialyzer membrane and into the dialysate. Dialysate exits the dialyzer **102** through dialyzer outlet **133** into fluid line **130**. Dialysate pump **131**, which can be located external to the manifolds, provides the driving force for pumping the dialysate through the dialysate flow path **101**. The dialysate can enter a first manifold **114** from fluid line **130** through manifold inlet **115**. The dialysate enters the manifold **114** into an internal conduit **117** of the manifold **114**. A valve **116** can selectively direct fluid into either internal conduit **122** or internal conduit **118**. In certain embodiments, a controller controls valve **116** to selectively direct the fluid in a flow path to a specified manifold outlet.

Internal conduit **118** is fluidly connected to manifold outlet **119** and fluid line **134**. An ultrafiltrate pump **120** can pump a portion of the fluid from valve **116**, through internal conduit **118** and fluid line **134** to either ultrafiltrate reservoir **121**, or alternatively, to a drain. In certain embodiments, the ultrafiltrate reservoir **121** can be positioned external to each of the manifolds, as illustrated in FIG. 1. The ultrafiltrate pump **120** and valve **116** can be in communication with the controller to control the amount of ultrafiltrate removed from the dialysate flow path **101**.

If needed, water from a water source **127** can be pumped by pump **124** through fluid line **126** and into an internal conduit **123** of manifold **114** through manifold inlet **125**. The water source **127** can be positioned external to each of the manifolds, as illustrated in FIG. 1. The water can be added to the dialysate flow path **101** in order to dilute the dialysate, or to provide a fluid bolus to the patient. The controller can be in communication with pump **124** to control the addition of water to the dialysate flow path **101** as necessary. The water can flow through internal conduit **123** to internal conduit **122** containing the dialysate in the dialysate flow path **101**. The diluted dialysate can exit the manifold **114** through manifold outlet **128** and into fluid line **129**.

Fluid line **129** can fluidly connect to a second manifold **103** at manifold inlet **104**. The dialysate can flow through manifold inlet **104** into internal conduit **111**. Valve **106** selectively directs fluid into either internal conduit **107** or internal conduit **109**. A conductivity sensor **105** can be included either inside or outside of the manifold **103** to determine the conductivity of the dialysate. In certain embodiments, conductivity sensor **105** can be in communication with the controller. If the conductivity as determined by conductivity sensor **105** indicates that the dialysate has solute concentrations outside of a predetermined range, the controller can control valve **106** to selectively direct fluid into internal conduit **109**. Internal conduit **109** is fluidly connected to manifold outlet **110** and fluid line **113**, which fluidly connects to fluid line **130** downstream of the dialyzer **102.** As such, if the dialysate is not safe or effective to pump through the dialyzer **102**, the controller can cause the fluid to bypass the dialyzer **102**, where additional solutes can be added or the dialysate can be diluted with water from water source **127**. Additional or alternative sensors (not shown), including temperature sensors, pressure sensors, pH sensors, and/or ammonia sensors can be included either inside or outside of the manifolds to determine one or more fluid parameters. If data from any of the sensors indicate an unsafe fluid parameter, the controller can control valve **106** to bypass dialyzer **102**.

If the sensors indicate that the dialysate is within safe ranges for all fluid parameters, the controller can control valve **106** to direct fluid into internal conduit **107**. Internal conduit **107** is fluidly connected to manifold outlet **108** and fluid line **112**. The dialysate can be pumped through fluid line **112** and through the dialyzer inlet **132** to contact blood from the patient.

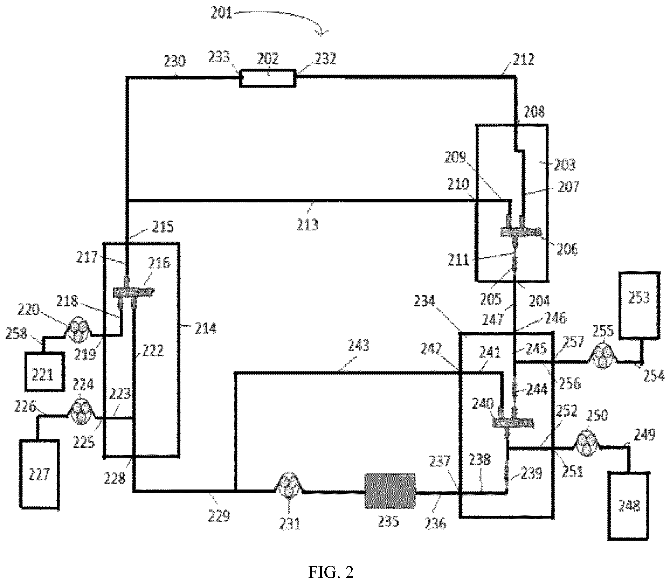

FIG. 2 illustrates a non-limiting dialysate flow path **201** with three integrated manifolds. The dialysate flow path **201** includes a fluid line **212** fluidly connected to a dialyzer inlet **232** of dialyzer **202.** As described, dialysate is pumped through the dialyzer **202** and exits the dialyzer **202** through dialyzer outlet **233** into fluid line **230**. Dialysate pump **231** provides the driving force for pumping the dialysate through the dialysate flow path **201**. The dialysate can enter a first manifold **214** from fluid line **230** through manifold inlet **215.** The dialysate enters the manifold **214** into an internal conduit **217**. A valve **216** can selectively direct fluid into either internal conduit **222** or internal conduit **218**. As described, a controller can control ultrafiltrate pump **220** to pump a portion of the fluid from internal conduit **218** through manifold outlet **219** and fluid line **258** into an ultrafiltrate reservoir **221**, or alternatively, to a drain. In certain embodiments, the ultrafiltrate reservoir **221** can be positioned external to the manifolds, as illustrated in FIG. 2, and will be considered to be a part of the system of integrated manifolds

Water from a water source **227** can be pumped by pump **224** through fluid line **226** and into an internal conduit **223** of manifold **214** through manifold inlet **225** to dilute the dialysate and/or provide a fluid bolus to the patient. The water source **227** can be positioned external to each of the manifolds, as illustrated in FIG. 2 and will be considered to be a part of the system of integrated manifolds. The controller can be in communication with pump **224** to control the addition of water to the dialysate flow path **201** as necessary. The water can flow through internal conduit **223** to internal conduit **222** containing the dialysate in the dialysate flow path **201**. The diluted dialysate can exit the manifold **214** through manifold outlet **228** and into fluid line **229**.

In certain embodiments, a sorbent cartridge **235** or other dialysate regeneration system can be employed to remove solutes and toxins from the dialysate, allowing the dialysate to be reused. The sorbent cartridge **235** can include one or more anion exchange resins, cation exchange resins, activated carbon, and urease. The urease catalyzes the conversion of urea in the dialysate to ammonium ions and carbonate ions. The ammonium ions can be subsequently captured by the cation exchange resin. Alternative methods of regenerating dialysate can be used in addition to, or in place of, sorbent cartridge **235**, including systems that use reverse osmosis, electrodialysis, or other methods known in the art.

After passing through sorbent cartridge **235**, the dialysate flows through fluid line **236** and into a second manifold **234** through manifold inlet **237** fluidly connected to internal conduit **238**. In certain embodiments, a conductivity sensor **239** can be positioned either inside or outside of the manifold **234** to determine the conductivity of the dialysate exiting sorbent cartridge **235**. Based on the determined conductivity bicarbonate can be added from bicarbonate concentrate source **248**. Pump **250** can pump a bicarbonate concentrate from bicarbonate concentrate source **248**, through fluid line **249** and into the second manifold **234** through manifold inlet **251**. Manifold inlet **251** can be fluidly connected to internal conduit **252**, which connects to internal conduit **238** to deliver the bicarbonate concentrate to the dialysate flow path **201**.

Valve **240** can selectively direct fluid from internal conduit **238** into either internal conduit **241** or internal conduit **245**. Internal conduit **241** is fluidly connected to manifold outlet **242** and fluid line **243**, which can be used to convey fluid with bicarbonate added to a point in the dialysate flow path **201** upstream of the sorbent cartridge **235**. By conveying a bicarbonate solution upstream of the sorbent cartridge **235** during priming of the system, the time required to prime the system can be reduced because the sorbent cartridge **235** need not be filled with water. During normal operation, the controller can control valve **240** to direct fluid into internal conduit **245.** An optional conductivity sensor **244** can be included to determine the conductivity of the dialysate after addition of bicarbonate to ensure that the bicarbonate concentration is within a predetermined range.

An infusate concentrate source **253** can contain a concentrated solution of one or more infusates, including magnesium, potassium, and calcium, each of which may be removed by the sorbent cartridge **235.** Infusate pump **255** can pump the infusate concentrate from the infusate concentrate source **253** through fluid line **254** and into the second manifold **234** through manifold inlet **257**. The infusate concentrate is pumped through internal conduit **256** to internal conduit **245**, where the infusate concentrate is mixed with the dialysate to generate a dialysate that can be safely pumped through dialyzer **202**. The dialysate can exit the second manifold **234** through manifold outlet **246** and flow through fluid line **247** to a third manifold **203** through manifold inlet **204.**

The dialysate can flow through manifold inlet **204** into internal conduit **211**. Valve **206** can selectively direct fluid into either internal conduit **207** or internal conduit **209**. A conductivity sensor **205** can be included either inside or outside of the third manifold **203** to determine the conductivity of the dialysate after addition of the infusates from infusate concentrate source **253.** Though shown as part of the third manifold **203**, one of skill in the art will understand that the conductivity sensor **205** can alternatively be positioned in manifold **234** or in fluid line **247** between the manifolds. If the conductivity as determined by conductivity sensor **205** indicates that the dialysate has solute concentrations outside of a predetermined range, the controller can control valve **206** to selectively direct fluid into internal conduit **209**, manifold outlet **210** and into fluid line **213** to bypass the dialyzer **202**. Additional or alternative sensors (not shown), including temperature sensors, flow sensors, pressure sensors, pH sensors, and/or ammonia sensors can be included either inside or outside of the manifolds, and can be used by the controller to determine whether valve **206** should be switched to bypass the dialyzer **202**. For example, if the temperature of the dialysate is too low, if ammonia is present in the dialysate, if the pH of the dialysate is too high or too low, the controller can automatically control valve **206** to selectively direct fluid to manifold outlet **208**, bypassing the dialyzer **202**. Flow sensors can determine the net movement of fluid across the dialyzer **202** and can be used to control valve **216** and ultrafiltrate pump **220** to control the net ultrafiltration from the patient. If the sensors indicate that the dialysate is within safe ranges for all fluid parameters, the controller can control valve **206** to direct fluid into internal conduit **207**, out of the third manifold **203** through manifold outlet **208** and into fluid line **212**.

Any number of integrated manifolds can be included in the dialysate flow path, including 2, 3, 4, 5, 6, 7, 8 or more integrated manifolds, each with manifold inlet and manifold outlets to selectively convey fluid in a specified flow path. Each of the integrated manifolds can include any number of manifold inlets and manifold outlets. As illustrated in FIG. 2, a manifold **203** can include one manifold inlet **204** and two manifold outlets **208** and **210**. In contrast, manifold **214** includes three manifold inlets **215**, **219**, and **225** and one manifold outlet **228**. Manifold **234** contains three manifold inlets **237**, **251**, and **257**, and two manifold outlets **242** and **246**. Alternative manifolds with 1, 2, 3, 4, 5, 6, or more manifold inlets and 1, 2, 3, 4, 5, 6, or more manifold outlets can be included and the three manifolds **203**, **214**, and **234** illustrated in FIG. 2 are for exemplary purposes only. In certain embodiments, additional manifolds can be included that can allow fluid to bypass the sorbent cartridge **235** in the case of ammonia breakthrough, or to otherwise control fluid movement in a specified flow path. In certain embodiments, all of the valves used in the system can be contained within the integrated manifolds, as illustrated in FIG.'s 1-2. Alternatively, one or more valves can be positioned on fluid lines external to the manifolds.

The system of integrated manifolds can include one or more fluid lines fluidly connecting the integrated manifolds, as illustrated in FIG.'s 1-2, or alternatively can include one or more manifolds connected directly together. A manifold outlet of a first manifold can be directly connected to a manifold inlet of a second manifold. For example, a manifold outlet of a first manifold can include a male portion of a connector, while a manifold inlet of a second manifold includes a female portion of a connector. The connectors on each manifold can be engaged to form a flow path directly from the first manifold to the second manifold, allowing fluid to be pumped through the dialysate flow path directly from the first manifold to the second manifold.

The connections between the manifold inlets, manifold outlets, and fluid lines can be any type of connections known in the art. In certain embodiments, a male portion of a connector can be provided on the manifold inlets and manifold outlets. The male portion of the connector can connect to a female portion at the ends of the fluid lines, or alternatively, can be inserted directly into the fluid lines. In certain embodiments, male portions of a connector can be included on the fluid lines and can be inserted into the manifold inlets and manifold outlets for connection to the internal conduits of the manifolds. Clamps or other means of securing the fluid lines to the manifold inlets and manifold outlets can be used in addition to, or as an alternative to, separate connectors.

One skilled in the art will understand that various combinations and/or modifications and variations can be made in the described systems and methods depending upon the specific needs for operation. Moreover features illustrated or described as being part of an aspect of the invention may be used in the aspect of the invention, either alone or in combination.

The invention may be described by reference to the following numbered paragraphs:-
1. A system of integrated manifolds; comprising:
   a dialysate flow path; the dialysate flow path fluidly connectable to a dialyzer inlet and a dialyzer outlet;
   the dialysate flow path comprising at least two manifolds;
   the at least two manifolds each comprising at least one internal conduit fluidly connecting a manifold inlet and a manifold outlet;
   a first fluid line fluidly connecting the dialyzer inlet to a first manifold;
   a second fluid line fluidly connecting the dialyzer outlet to a second manifold; and
   at least one fluid line fluidly connecting a manifold outlet of a least one manifold to a manifold inlet of at least one other manifold.
2. The system of integrated manifolds of paragraph 1, wherein the dialysate flow path comprises at least three manifolds.
3. The system of integrated manifolds of paragraph 1, wherein at least one manifold comprises at least one valve fluidly connecting the at least one internal conduit to either a first and second manifold outlet or a first and second manifold inlet.
4. The system of integrated manifolds of paragraph 3, the at least one valve in electronic communication with a controller; the controller controlling the at least one valve to selectively direct fluid through at least one manifold from a specified inlet to a specified outlet.
5. The system of integrated manifolds of paragraph 1, wherein at least one manifold comprises at least a first manifold outlet and a second manifold outlet fluidly connected to the at least one internal conduit; and
   at least one valve fluidly connecting the at least one internal conduit to the first manifold outlet and the second manifold outlet.
6. The system of integrated manifolds of paragraph 1, wherein at least one manifold comprises at least a first manifold inlet and a second manifold inlet fluidly connected to the at least one internal conduit.
7. The system of integrated manifolds of paragraph 1, wherein at least one manifold comprises at least a first internal conduit and a second internal conduit; wherein either both the first internal conduit and the second internal conduit are fluidly connected to a manifold inlet, or wherein both the first internal conduit and the second internal conduit are fluidly connected to a manifold outlet.
8. The system of integrated manifolds of paragraph 7, wherein the first internal conduit is fluidly connected to the dialyzer inlet; and wherein the second internal conduit is fluidly connected to the dialyzer outlet.
9. The system of integrated manifolds of paragraph 1, wherein at least one manifold comprises at least one sensor.
10. The system of integrated manifolds of paragraph 9, wherein the at least one sensor is selected from the group consisting of: a pressure sensor, a conductivity sensor, a temperature sensor, a flow sensor, and combinations thereof.
11. The system of integrated manifolds of paragraph 1, wherein the dialysate flow path comprises at least one dialysate pump external to a manifold.
12. The system of integrated manifolds of paragraph 1, wherein the dialysate flow path comprises a sorbent cartridge external to a manifold.
13. The system of integrated manifolds of paragraph 1, wherein all valves within the dialysate flow path are positioned within one or more manifolds.
14. A method of using the system of integrated manifolds of paragraph 1; comprising the steps of:
   pumping a dialysate from the dialyzer outlet through a first manifold;
   pumping the dialysate from the first manifold through a second manifold; and
   pumping the dialysate from the second manifold to the dialyzer inlet.
15. The method of paragraph 14, further comprising the step of controlling one or more valves within at least one manifold to selectively direct fluid from a specified manifold inlet to a specified manifold outlet.
16. The method of paragraph 15, wherein the step of controlling one or more valves is performed by a controller in electronic communication with the one or more valves.
17. The method of paragraph 14, further comprising the step of determining at least one fluid parameter of the dialysate with at least one sensor located within at least one manifold.
18. The method of paragraph 17, further comprising the step of controlling one or more valves within at least one manifold to selectively direct fluid from a specified manifold inlet to a specified manifold outlet based on the at least one fluid parameter.
19. The method of paragraph 18, wherein the at least one fluid parameter comprises a conductivity.
20. The method of paragraph 18, further comprising the step of controlling at least one pump to direct a fluid from a concentrate source to the at least one internal conduit based on the at least one fluid parameter.
21. The method of paragraph 14, further comprising the step of pumping a portion of the dialysate to an ultrafiltrate reservoir; wherein the ultrafiltrate reservoir is external to the manifolds.
22. The method of paragraph 14, further comprising the step of pumping water from a water source to the dialysate flow path; wherein the water source is external to the manifolds.
23. The system of integrated manifolds of paragraph 1, further comprising an ultrafiltrate reservoir fluidly connected to the dialysate flow path; the ultrafiltrate reservoir external to the at least two manifolds.
24. The system of integrated manifolds of paragraph 1, further comprising a water source fluidly connected to the dialysate flow path; the water source external to the at least two manifolds.

## Claims

1. A system of integrated manifolds; comprising:
a dialysate flow path; the dialysate flow path fluidly connectable to a dialyzer inlet and a dialyzer outlet;
the dialysate flow path comprising at least two manifolds;
the at least two manifolds each comprising at least one internal conduit fluidly connecting a manifold inlet and a manifold outlet;
a first fluid line fluidly connecting the dialyzer inlet to a first manifold;
a second fluid line fluidly connecting the dialyzer outlet to a second manifold; and
at least one fluid line fluidly connecting a manifold outlet of a least one manifold to a manifold inlet of at least one other manifold.

2. The system of integrated manifolds of claim 1, wherein the dialysate flow path comprises at least three manifolds.

3. The system of integrated manifolds of claim 1 or claim 2, wherein at least one manifold comprises at least one valve fluidly connecting the at least one internal conduit to either a first and second manifold outlet or a first and second manifold inlet.

4. The system of integrated manifolds of claim 3, the at least one valve in electronic communication with a controller; the controller controlling the at least one valve to selectively direct fluid through at least one manifold from a specified inlet to a specified outlet.

5. The system of integrated manifolds of claim 1, wherein at least one manifold comprises at least a first manifold outlet and a second manifold outlet fluidly connected to the at least one internal conduit; and
at least one valve fluidly connecting the at least one internal conduit to the first manifold outlet and the second manifold outlet.

6. The system of integrated manifolds of claim 1, wherein at least one manifold comprises at least a first manifold inlet and a second manifold inlet fluidly connected to the at least one internal conduit.

7. The system of integrated manifolds of claim 1, wherein at least one manifold comprises at least a first internal conduit and a second internal conduit; wherein either both the first internal conduit and the second internal conduit are fluidly connected to a manifold inlet, or wherein both the first internal conduit and the second internal conduit are fluidly connected to a manifold outlet.

8. The system of integrated manifolds of claim 7, wherein the first internal conduit is fluidly connected to the dialyzer inlet; and wherein the second internal conduit is fluidly connected to the dialyzer outlet.

9. The system of integrated manifolds of any preceding claim , wherein at least one manifold comprises at least one sensor.

10. The system of integrated manifolds of claim 9, wherein the at least one sensor is selected from the group consisting of: a pressure sensor, a conductivity sensor, a temperature sensor, a flow sensor, and combinations thereof.

11. The system of integrated manifolds of any preceding claim , wherein the dialysate flow path comprises one or more of the following:-
(i) at least one dialysate pump external to a manifold;
(ii) a sorbent cartridge external to a manifold;
(iii) all valves within the dialysate flow path positioned within one or more manifolds.

12. A method of using the system of integrated manifolds of any preceding claim ; comprising the steps of:
pumping a dialysate from the dialyzer outlet through a first manifold;
pumping the dialysate from the first manifold through a second manifold; and
pumping the dialysate from the second manifold to the dialyzer inlet, optionally further comprising the step of controlling one or more valves within at least one manifold to selectively direct fluid from a specified manifold inlet to a specified manifold outlet,
and optionally wherein the step of controlling one or more valves is performed by a controller in electronic communication with the one or more valves.

13. The method of claim 12, further comprising the step of determining at least one fluid parameter of the dialysate with at least one sensor located within at least one manifold, and optionally further comprising the step of controlling one or more valves within at least one manifold to selectively direct fluid from a specified manifold inlet to a specified manifold outlet based on the at least one fluid parameter,optionally wherein the at least one fluid parameter comprises a conductivity.

14. The method of claim 13, further comprising the step of controlling at least one pump to direct a fluid from a concentrate source to the at least one internal conduit based on the at least one fluid parameter.

15. The method of any of claims 12-14, further comprising the step of pumping a portion of the dialysate to an ultrafiltrate reservoir; wherein the ultrafiltrate reservoir is external to the manifolds, and optionally further comprising the step of pumping water from a water source to the dialysate flow path; wherein the water source is external to the manifolds.
